# EUROPÄISCHE PATENTSCHRIFT

(11) **EP 1 126 805 B1**
(45) Veröffentlichungstag und Bekanntmachung des Hinweises auf die Patenterteilung: **17.01.2007**
(21) Anmeldenummer: 99971692.1
(22) Anmeldetag: 05.11.1999
(51) Int. Cl.: A61F 9/011

(54) **MEDIZINISCHES INSTRUMENT ZUR PHACOEMULSIFIKATION**
MEDICAL INSTRUMENT FOR PHACOEMULSIFICATION
INSTRUMENT MEDICAL POUR PHACO-EMULSIFICATION

(30) Priorität: 06.11.1998 DE 19852574
(43) Veröffentlichungstag der Anmeldung: 29.08.2001
(73) Patentinhaber: Carl Zeiss Meditec AG, 07745 Jena (DE)
(72) Erfinder: ELBRECHT, Jens, D-07747 Jena (DE); SCHRÖDER, Eckhard, D-90542 Eckental (DE); WEIDNER, Frank, D-07751 Löberschütz (DE)
(74) Vertreter: Geyer, Fehners & Partner
(86) Internationale Anmeldenummer: PCT/EP1999/008472
(87) Internationale Veröffentlichungsnummer: WO 2000/027325

(56) Entgegenhaltungen:
- WO-A-93/20895
- DE-A- 3 822 011
- US-A- 3 844 272
- US-A- 4 002 169
- US-A- 5 257 988

## Beschreibung

### Gebiet der Erfindung

Die Erfindung bezieht sich auf ein medizinisches Instrument zur Phacoemulsifikation, mit dem das biologische Gewebe einer Augenlinse durch Energieeintrag zerkleinert, das Zerkleinerungsprodukt durch einen Schnitt in der Augenhornhaut abgesaugt und dabei der intraokulare Druck durch Zufuhr einer Irrigationsflüssigkeit aufrechterhalten wird.

### Stand der Technik

Zum Zwecke der Ausführung der vorgenannten Operation verfügen die im Stand der Technik bekannten Instrumente über eine Zuführkanüle für die Irrigationsflüssigkeit und eine Absaugkanüle für den Kern bzw. für Kernfragmente. Beim klassischen Verfahren der Phacoemulsifikation wird eine Kanüle in das Auge eingeschoben, um den kartaraktösen Linsenkern insgesamt abzusaugen. Diese seit ihrer Begründung im Jahre 1967 bekannt Methode ist allerdings lediglich für weiche Linsen vorteilhaft anwendbar.

Bei härteren Kernen wird durch Zuführung von Energie, vorzugsweise über Ultraschall oder Laser, zunächst eine Zerkleinerung des Linsenmaterials vorgenommen und die kleinen Bruchstücke dann durch die Absaugkanüle, die von einem Handstück umschlossen ist, abgesaugt. Das aus der Vorkammer abgesaugte Volumen an Flüssigkeit und Linsenbruchstücken wird kompensiert, indem über die Zuführkanüle Spülflüssigkeit in die Vorkammer infundiert wird, wodurch der intraokulare Druck im wesentlichen erhalten bleibt.

Die Zuführung der Flüssigkeit, als Irrigation bezeichnet, erfolgt bei allen bekannten Handstücken über eine Zuführkanüle, die als über die Absaugkanüle gestülpte Hülse ausgebildet ist. Während der Operation fließt die Flüssigkeit zwischen Absaugkanüle und Hülseninnenwand zum distalen Ende des Handstückes, wo in der Regel zwei Austrittsöffnungen vorgesehen sind.

Der Energieeintrag erfolgt derzeit bei über 90% der Kataraktextraktionen mit Hilfe von Ultraschall. Nachteiligerweise ist das Einbringen von Ultraschall in das Gewebe mit einer unerwünschten Wärmeentwicklung verbunden, so daß, um Schäden am gesunden Gewebe zu vermeiden, gekühlt werden muß. Insofern kommt bei der Ultraschall-Phacoemulsifikation der Hülse eine Doppelrolle zu, indem die zwischen Absaugkanüle und Hülseninnenwand fließende Flüssigkeit zugleich zur Kühlung genutzt wird. Aus diesem Grund werden Handstücke zur Phacoemulisfikation mit Hilfe der Ultraschalltechnik grundsätzlich nur mit integrierter Zuführkanüle, Absaugkanüle und integriertem Ultraschalleiter ausgeführt.

Aufgrund der Notwendigkeit der Zuführung von Flüssigkeit und der Ausbildung der Hülse als Kühlelement wird das distale Ende des Handstücks notwendigerweise verhältnismäßig groß. So verdickt sich beispielsweise die Absaugkanüle, die an sich einen Durchmesser im Bereich zwischen 0,8 mm und 1,3 mm aufweist, durch den Einsatz der darübergezogenen Hülse auf ca. 2,5 mm Durchmesser. Um eine Kanüle dieser Abmessung in das Gewebe einbringen zu können, ist ein Schnitt in der Grö-ßenordnung von 3 mm bis 3,2 mm notwendig.

Derart große Schnitte allerdings haben Verluste von Kammerwasser und von Spüllösung während der Operation zur Folge. Außerdem wird das Risiko eines durch die Operation induzierten Astigmatismus um so größer, je größer der Schnitt ist. Auch verläuft die visuelle Rehabilitation bei Schnitten solcher Größe verhältnismäßig langsam im Vergleich zu Schnitten geringerer Größe.

Aus diesem Grund geht das Bestreben der Medizin zu immer kleineren Schnitten mit der Maßgabe, durch diese kleinen Schritte injezierbare Linsen in die Vorderkammer einzubringen. Das allerdings setzt voraus, daß durch kleine Schnitte sowohl das zerkleinerte Linsenmaterial abgesaugt als auch die Irrigationsflüssigkeit zugeführt werden kann.

Im Stand der Technik ist weiterhin die Laser-Phacoemulsifikation bekannt, bei der der Energieeintrag über Laserstrahlung erfolgt, die direkt auf das zu zerkleinernde Gewebe gerichtet wird. Dabei muß eine Laserwellenlänge mit sehr geringer Eindringtiefe in das wässrige Material des Augeninnern gewählt werden, wofür Wellenlängen im tiefen UV oder im Infrarotbereich geeignet sind. In diesem Zusammenhang hat sich der Einsatz von Er:YAG-Lasern mit einer Wellenlänge um λ = 2,9 µm bewährt. Auf diese Weise wird ein quasi nichtthermischer Energieeintrag erzielt und die Probleme, die mit der Verwendung von Ultraschall im Hinblick auf die Wärmeentwicklung verbunden sind, treten nicht mehr auf.

Allerdings weisen die im Stand der Technik bekannten Handstücke zur Laser-Phacoemulsifikation ebenfalls den Nachteil auf, daß Zuführkanüle, Absaugkanüle und die Energiezuführung (beispielsweise über Lichtleitfaser) in ein Handstück integriert sind und die Mündungen örtlich auf ein gemeinsames Areal ausgerichtet sind. Insofern ist auch hier ein großer Schnitt erforderlich, durch den die Zuführung der Irrigationsflüssigkeit und der Laserenergie und auch die Absaugung des zerkleinerten Kernmaterials vorgenommen werden muß.

*In DE 38 22 011 A ist ein augenchirurgisches Instrument zum Absaugen von Linsenresten und ein Laser-Phaco-Kataraktabsaugsystem mit zwei derartigen Instrumenten beschrieben. Das augenchirurgische Instrument besitzt ein Handstück mit einer Kammer, von der ausgehend sich eine Kanüle erstreckt und in die je eine Saugleitung, eine Zuführleitung und, fluchtend mit der Kanüle, eine Führung für einen Lichtleiter einmünden. Der Lichtleiter ist in der Kanüle verschiebbar und in einer jeweils gewählten Einstellage fixierbar. Das ebenfalls beschriebene Laser-Phaco-Kataraktabsaugsystem ist mit zwei derartigen Instrumenten ausgerüstet und insofern für eine bimanuelle Operationstechnik ausgelegt. Dabei sind die Saugleitungen und die Zuführleitungen jeweils über umschaltbare Ventile mit einer Vakuum-Pumpe bzw. einer Spülflüssigkeitsquelle verbunden.*

### Beschreibung der Erfindung

Davon ausgehend besteht die Aufgabe der Erfindung darin, ein medizinisches Instrument zur Laser-Phacoemulsifikation zu schaffen, mit dem unter Beibehaltung des vorteilhaften quasi nichtthermischen Energieeintrages mittels Laserstrahlung nicht nur das Entfernung der Linse, sondern auch das Ersetzen der Linse durch kleine Schnitte in der Hornhaut hindurch möglich ist.

Die Aufgabe wird erfindungsgemäß bei einem medizinischen Instrument der vorgenannten Art mit den in den Ansprüchen 1 bis 20 angegebenen Merkmalen gelöst.

Mit dem erfindungsgemäß vorgeschlagenen medizinischen Instrument ist es nicht nur möglich, die Phakoemulsifikation durch kleine Schnitte hindurch auszuführen, woraus sich im wesentlichen die Vorteile ergeben, daß nur noch ein geringes Risiko im Hinblick auf die Induzierung eines Astigmatismus besteht und auch eine schnellere visuelle Rehabilitation nach der Operation möglich ist, sondern es wird auch eine Phakoemulsifikationstechnik zugänglich, bei der durch Injektion eine Linse in das Auge eingebracht werden kann.

Insofern besteht eine sehr bevorzugte Anwendung des erfindungsgemäß vorgeschlagenen Instrumentes darin, nach Absaugung des zerkleinerten Kernmaterials über eines der beiden Handstücke bzw. über die in das jeweilige Handstück integrierte Kanüle die Substanz zu injizieren, die dann im Auge verbleibt und als neue Augenlinse die alte ersetzt.

Hierzu sind in Ausgestaltungsvarianten der Erfindung die Handstücke bzw. die Kanülen mit Anschlüssen für entsprechende Geräte zum Einbringen einer solchen Substanz, etwa für Spritzen o.ä., ausgestattet.

In einer weiteren bevorzugten Ausgestaltungsvariante ist jedes der beiden Handstücke sowohl zum Absaugen des zerkleinerten Kernmaterials auch zur Zuführung der Spülflüssigkeit vorgesehen. Werden beispielsweise die Kanülen beider Handstücke mit ihrem distalen Ende von gegenüberliegenden Seiten zur Augenlinse vorgeschoben, ist es auf diese Weise möglich, an diesen beiden sich gegenüberliegenden Positionen wahl- und wechselweise sowohl die Zerkleinerung des Gewebes als auch die Absaugung der Ablationsprodukte vorzunehmen.

Das hat gegenüber der einseitigen Absaugung zum Vorteil, daß die Kanüle weniger tief eingeführt werden muß, was wiederum dem Bestreben nach kleinen Schnitten und schonender Gewebsbehandlung entgegenkommt. Zu diesem Zweck sind beide Handstücke über Förderleitungen mit einem umschaltbaren Wegeventil verbunden, mit dessen Hilfe die Strömungsrichtungen bei der Absaugung des Ablationsproduktes und bei der Zuführung der Spülflüssigkeit umgekehrt werden können. Diese Umschaltung ist vorteilhaft mit einem Fußschalter ausführbar.

In diesem Zusammenhang kann weiterhin vorgesehen sein, daß beide Handstücke mit Fasertips zur Zuführung der Laserenergie ausgestattet sind. Die Fasertips können vorzugsweise innerhalb der Kanüle angeordnet sein und auch innerhalb der Kanüle in Einstrahlungsrichtung der Laserenergie bzw. in Strömungsrichtung verschiebbar angeordnet sein. Damit ergibt sich der Vorteil, daß der Fasertip beispielsweise dann vom Behandlungsort zurückgezogen werden kann, wenn während der Irrigation keine Einstrahlung von Laserenergie erfolgt. Das Austrittsende der betreffenden Kanüle verbleibt dabei im Gegensatz zum Fasertip unmittelbar am Behandlungsort.

In einer weiterhin sehr bevorzugten Ausgestaltung der Erfindung ist vorgesehen, einem oder auch beiden der Handstücke eine Einrichtung zur Beseitigung von Verstopfungen innerhalb der Kanüle oder innerhalb der Förderleitungen zuzuordnen. Diese Einrichtung kann beispielsweise über ein seitlich in die Kanüle und/oder in eine der Förderleitungen einführbares und innerhalb des jeweiligen Leitungsquerschnittes verschiebbares Reinigungselemente verfügen, wie beispielsweise ein mit verdicktem Ende ausgestatteter flexibler Stab oder eine Reinigungsspirale, deren Durchmesser dem Leitungsquerschnitt angepaßt sind.

Eine weitere vorteilhafte Ausgestaltung besteht darin, daß eines oder auch beide der Handstücke mit Hilfsinstrumenten zum Halten, Greifen, Drehen, Schieben, Schneiden Spalten, Schützen und/oder zum Beleuchten des Gewebes am Behandlungsort ausgestattet sind. Vorzugsweise sind diese Hilfselemente gegeneinander austauschbar und beispielsweise über einen Aufsteckmechanismus mit dem jeweiligen Handstück verbindbar. In diesem Zusammenhang sind weitere Ausgestaltungen denkbar, bei denen die Hilfsinstrumente über einen Verschiebemechanismus im Handstück versenkbar sind oder auch außen am Handstück verschiebbar angeordnet sind, so daß sie bei Bedarf jederzeit in die entsprechende Gebrauchsposition gebracht werden können.

### Kurze Beschreibung der Zeichnungen

Die Erfindung soll nachfolgend anhand zweier Ausführungsbeispiele näher erläutert werden. In den zugehörigen Zeichnungen zeigen:
- Fig.1: die Prinzipdarstellung eines ersten Ausführungsbeispieles
- Fig.2: eine Ausschnittsvergrößerung aus Fig.1
- Fig.3: die Prinzipdarstellung eines zweiten Ausführungsbeispieles
- Fig.4: eine Auswahl an Hilfsinstrumenten

### Ausführliche Beschreibung der Zeichnungen

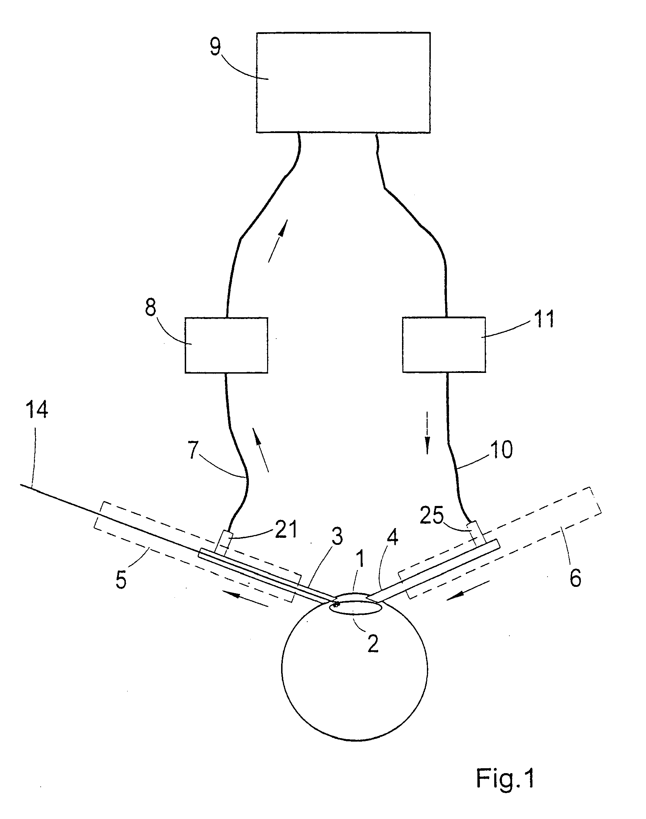

In einem ersten Ausführungsbeispiel nach Fig.1 ist symbolisch eine von einer Hornhaut 1 verdeckte Augenlinse 2 dargestellt, die mit Hilfe der erfindungsgemäßen Anordnung extrahiert werden soll. Zu diesem Zweck sind aus etwa entgegengesetzten Richtungen eine Kanüle 3 und eine Kanüle 4 durch Schnitte in der Hornhaut 1 hindurch bis zur Augenlinse 2 in die Vorkammer des Auges eingeführt. Weiterhin ist in Fig.1 zu erkennen, daß die Kanüle 3 einem Handstück 5 und die Kanüle 4 einem Handstück 6 zugeordnet sind.

Des weiteren zeigt Fig.1, daß das Handstück 5 und mit diesem die Kanüle 3 über eine Förderleitung 7, beispielsweise einem flexiblen Schlauch, und über eine Absaugpumpe 8 mit einer Irrigations-/Aspirationseinheit 9 verbunden ist. In analoger Weise besteht eine Verbindung des Handstückes 6 und damit der Zuführkanüle 4 über eine Förderleitung 10 zu einer Infusionspumpe 11, die ihrerseits mit der Irrigations-/Aspirationseinheit 9 gekoppelt ist.

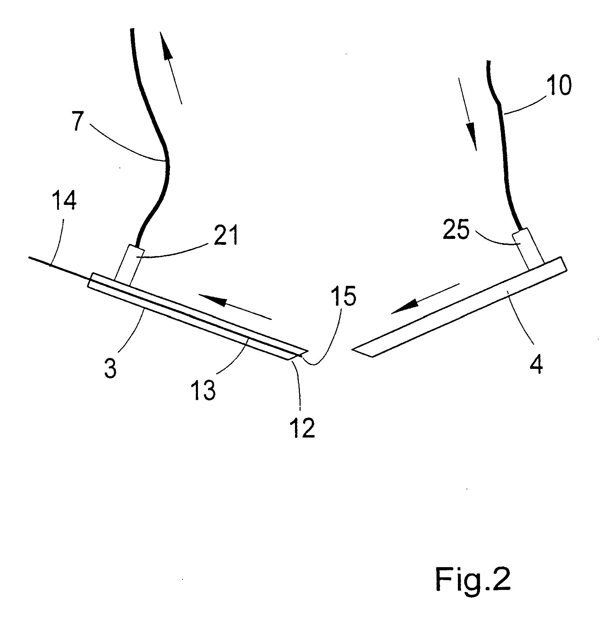

Die Kanüle 3 weist, wie in Fig.2 vergrößert dargestellt ist, eine Mündungsöffung 12 auf, die um einen Winkel α ≠ 90° gegen die Absaugrichtung geneigt ist. Von der Mündungsöffnung 12 umschlossen ist ein Fasertip 13, der über eine innerhalb des Handstückes 5 vorgesehene Koppeloptik (zeichnerisch nicht dargestellt) mit einer Lichtleitfaser 14 verbunden ist, die ihrerseits an eine (zeichnerisch ebenfalls nicht dargestellte) Lasereinheit angeschlossen ist. Dieser enthält vorzugsweise einen Er:YAG-Laser als Strahlungsquelle. Über die Lichtleitfaser 14 wird die Laserstrahlung von der Laserquelle herangeführt und steht an der Lichtaustrittsfläche 1 5 des Fasertips 13 zur Einstrahlung in das Kernmaterial zur Verfügung.

Die Lichtaustrittsfläche 15 kann rechtwinklig zur Ansaugrichtung ausgerichtet sein; denkbar ist jedoch auch, die Lichtaustrittfläche 1 5 gegen die Absaugrichtung geneigt auszuführen.

Bei Beginn der Kataraktextraktion wird zunächst die schräg angeschliffene Mündungsöffnung 12 der Kanüle 3 in die Vorkammer des Auges eingeführt, dann die Irrigations-/Aspirationseinheit 9 angesteuert, wodurch die Ansaugpumpe 8, die Infusionspumpe 11 und die Lasereinheit in Betrieb genommen werden. Dabei wird Laserenergie in das Kernmaterial eingestrahlt und dieses abschnittweise zerkleinert. Die Bruchstückchen werden durch die Mündungsöffnung 13, die Absaugkanüle 3 und die Förderleitung 7 hindurch abgesaugt.

Um zu vermeiden, daß mit der Absaugung der Flüssigkeit und der Linsenbruchstücke aus der Vorkammer der intraokulare Druck absinkt, wird durch die inzwischen von der Gegenseite eingeführte 4 Spülflüssigkeit infundiert, die von der lrrigations-/Aspirationseinheit 9 über die Infusionspumpe 11 und die Förderleitung 10 bereitgestellt wird. Mit der Flüssigkeitszufuhr wird der Druck innerhalb der Vorkammer aufrecht erhalten.

Aufgrund der erfindungsgemäßen lokalen Trennung der Kanüle 3 und der Kanüle 4 ist es möglich, beide Kanülen 3,4 mit einem Durchmesser von maximal 1,3 mm auszuführen. Da mit der Einstrahlung der Laserenergie im Gegensatz zur Ultraschall-Phacoemulsifikation ein quasi nichtthermischer Prozeß abläuft, muß die Irrigationsflüssigkeit nicht zur Kühlung verwendet werden und kann demzufolge räumlich getrennt von der Kanüle 3 zugeführt werden.

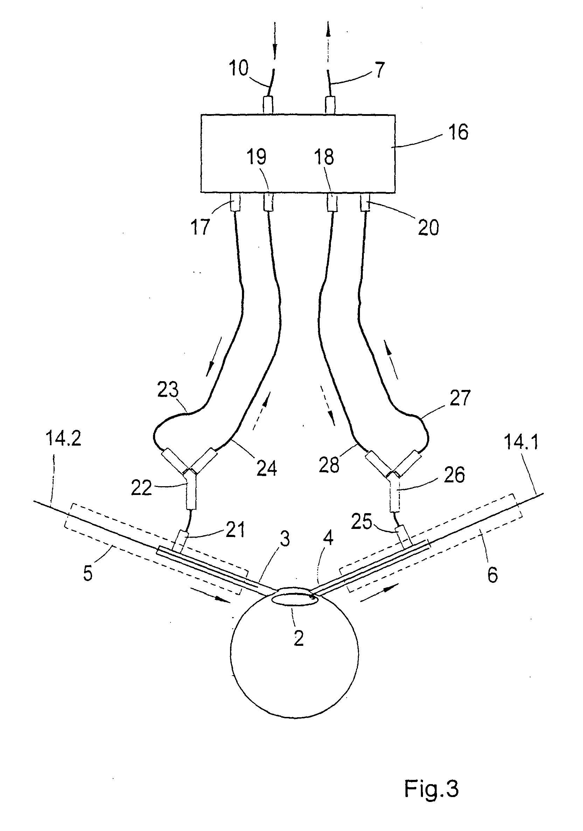

In einem zweiten Ausführungsbeispiel, das im folgenden anhand Fig.3 erläutert werden soll, ist vorgesehen, daß in die Förderleitungen zwischen den Handstücken 5 und 6 und der Irrigations-/Aspirationseinheit 9 ein umschaltbares Wegeventil 1 6 eingeordnet ist. Dieses Wegeventil 16 ist einerseits mit der von der Infusionspumpe 11 kommenden Förderleitung 10 und der zur Absaugpumpe 8 führenden Förderleitung 7 verbunden. Andererseits verfügt das Wegeventil 16 über zwei Ausgänge 17 und 18 sowie zwei Eingänge 19 und 20.

Wie weiterhin in Fig.3 dargestellt ist, ist das Handstück 5 mit dem Ausgang 17 und auch mit dem Eingang 19 verbunden, analog das Handstück 6 mit dem Ausgang 18 und dem Eingang 20. Der Anschluß 21 am Handstück 5 ist über eine Verzweigung 22 in zwei Leitungswege aufgezweigt, von denen einer über eine Förderleitung 23 mit dem Ausgang 17 und ein zweiter über eine Förderleitung 24 mit dem Eingang 19 verbunden ist.

In derselben Weise ist der Anschluß 25 des Handstückes 6 über eine Verzweigung 26 in zwei Leitungswege aufgezweigt, von denen einer über eine Förderleitung 27 mit dem Eingang 20 und ein zweiter über eine Förderleitung 28 mit dem Ausgang 18 verbunden ist.

Das Wegeventil 16 ist mit einem (zeichnerisch nicht dargestellten) Fußschalter verbunden, mit dessen Hilfe das Wegeventil 16 so umgeschaltet werden kann, daß der Zufluß von der Förderleitung 10 wahlweise am Ausgang 17 oder am Ausgang 18 liegt und die Förderleitung 7 für die Absaugung wahlweise mit dem Eingang 19 oder dem Eingang 20 verbunden ist.

Wird nun eine erste dieser beiden möglichen Ventilstellungen gewählt und der Fußschalter entsprechend betätigt, ist der Ausgang 17 über die Förderleitung 23 mit dem Handstück 5 verbunden und es wird über die Kanüle 3 Spülflüssigkeit zugeführt. Zugleich ist in dieser ersten Ventilstellung das Handstück 6 über die Verzweigung 26 und die Förderleitung 27 mit dem Eingang 20 verbunden, wobei durch die Kanüle 4 hindurch das abladierte Kernmaterial abgesaugt wird und über das Wegeventil 16 und die Förderleitung 7 zur Absaugpumpe 8 gelangt. Die Zuführung der Laserenergie erfolgt dabei durch einen innerhalb der Kanüle 4 vorhandenen Fasertip 14.1.

Hat der Operateur die Absicht, das Kernmaterial von der gegenüberliegenden Seite der Augenlinse her zu bearbeiten, besteht die Möglichkeit, den Fußschalter zu betätigen und damit das Wegeventil 16 in die zweite Ventilstellung zu bringen, in welcher nun die Durchflußverbindung zwischen der Förderleitung 10 und dem Ausgang 17 getrennt und dafür die Verbindung zwischen der Förderleitung 10 und dem Ausgang 18 hergestellt wird. Ebenso wird die Durchflußverbindung zwischen dem Eingang 20 und der Förderleitung 7 getrennt und dafür die Verbindung zwischen dem Eingang 19 und der Förderleitung 7 hergestellt.

Nunmehr erfolgt die Zuführung der Spülflüssigkeit von der Infusionspumpe über die Förderleitung 10 durch das Wegeventil 16 hindurch sowie über dessen Ausgang 18 durch die Förderleitung 28 und die Verzweigung 26 in das Handstück 6 bzw. in die im Handstück 6 integrierte Kanüle 4 und von deren distalen Ende zum Behandlungsort.

Zugleich erfolgt die Absaugung mit dem Handstück 5, da nun dessen Kanüle 3 über die Verzweigung 22, die Förderleitung 24, den Eingang 19, das Wegeventil 16 und die Förderleitung 7 mit der Absaugpumpe 8 verbunden ist.

In diesem Zusammenhang kann nun vorgesehen sein, daß auch dem Handstück 5 ein Fasertip zur Zuführung von Laserenergie zugeordnet und innerhalb der Kanüle 3 angeordnet ist. Dieser kann beispielsweise über die Lichtleitfaser 14.2 mit der Laserquelle verbunden sein. Damit kann auch über das Handstück 5 Energie in das Kernmaterial eingebracht, das Kernmaterial abladiert und durch die Kanüle 3 hindurch wie beschrieben abgesaugt werden.

Wird der Fasertip im jeweiligen Handstück 5, 6 zeitweilig nicht benutzt, kann er gemäß einer Option der Erfindung in die betreffenden Kanäle 3, 4 zurückgezogen werden, wie das beispielhaft in der Kanüle 3 angedeutet ist. Dadurch wird das Einbringen der Spülflüssigkeit nicht behindert.

Unter Umständen ist es vorteilhaft, wenn die Verzweigungen 22, 26 mit Rückschlagventilen ausgestattet sind, durch die verhindert wird, daß während des Umschaltens des Wegeventiles 16 oder unmittelbar nach der Umschaltung bereits abgesaugte Flüssigkeit unbeabsichtigt wieder zum Auge gelangt. Die Rückschlagventile sind im einfachsten Falle als frei bewegliche Klappen ausgeführt, die sich bei Flüssigkeitsstau oder Rückfluß dichtend auf Ventilsitze legen und so den Rückfluß verhindern.

In bevorzugten Ausgestaltungsvarianten kann vorgesehen sein, eines der beiden Handstücke oder unter Umständen auch beide Handstücke mit mechanischen Hilfsinstrumenten, beispielsweise zur Fixierung, Mobilisierung oder Teilung der Linse oder von Linsenteilen, zu gekoppeln. Damit müssen für diese Zwecke keine separaten Hilfsinstrumente in die Vorkammer eingebracht werden, sondern die erforderlichen Handlungen sind mit den so aufgerüsteten Handstücken ausführbar.

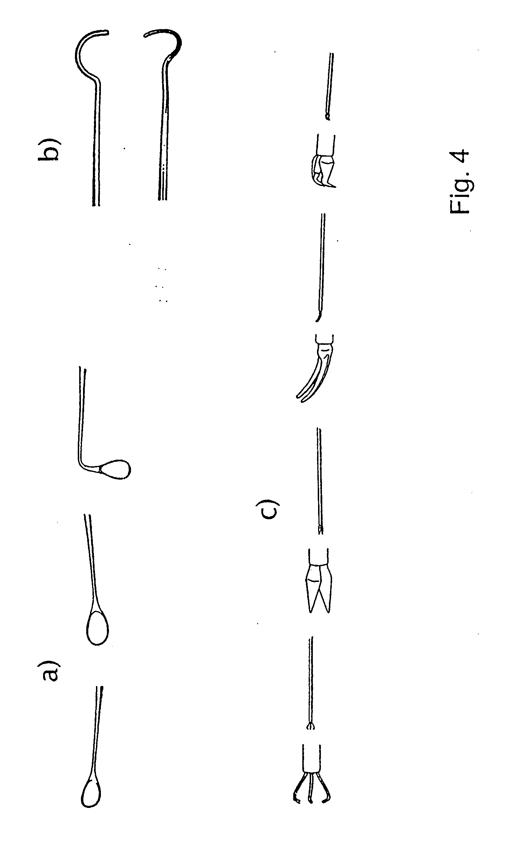

Eine Auswahl von Hilfsinstrumenten zeigt Fig.4. Hier sind unter Fig.4a) beispielsweise Schlingen zum Halten, unter Fig.4b) Haken zum Halten und unter Fig.4c) Hilfsinstrumente zum Greifen dargestellt. Weiterhin können Hilfsinstrumente zum Schneiden (Scheren) und/oder zum Spalten des Kernmaterials (Messer), zum Schützen (Schutzschilde) oder auch zum Beleuchten (Faserenden) vorgesehen sein. Derartige Hilfsinstrumente sind im Stand der Technik bekannt und müssen deshalb an dieser Stelle nicht näher beschrieben werden.

Diese Technik der Phacoemulsifikation, die mit dem erfindungsgemäßen Instrument anwendbar ist, hat vorteilhaft zur Folge, daß die Schnitte in die Hornhaut wesentlich kleiner ausgeführt werden können als beim Stand der Technik. Damit werden geringere Verluste von Kammerwasser und Spüllösung während der Operation erzielt, woraus sich neben dem geringeren Bedarf an Spüllösung des weiteren auch ein reduziertes Flüssigkeitsvolumen und damit eine Schonung der Gewebsflächen der Vorkammer, insbesondere der Endothelschicht der Hornhaut, ergibt. Au-ßerdem ist aufgrund der kleinen Schnitte das Risiko geringer, durch die Operation einen Astigmatismus zu induzieren. Auch ist bei Schnitten dieser Größe eine schnellere visuelle Rehabilitation nach der Operation möglich.

Die erfindungsgemäße Ausführung des medizinischen Instrumentes zur Laser-Phacoemulsifikation mit zwei Handstücken ermöglicht eine bimanuelle Phakotechnik, die neben den vorgenannten Vorteilen auch dem Operateur eine höhere Flexibilität im intraoperativen Handling gestattet. Mit dem Emulsifikationsprinzip, das mit diesem Instrument ausführbar ist, kann der Operateur den Katarakt durch wesentlich kleinere Schnitte in der Hornhaut entfernen als das im Stand der Technik möglich war. Damit kann die Linse im Kapselsack liegend emulsifiziert und abgesaugt werden, ein Eröffnung des Kapselsackes durch Kapsulorhexis ist nun nicht mehr erforderlich.

Wie weiter oben dargestellt, ist es mit diesem Handstück möglich, die operative Maßnahme am Auge bei wesentlich kleineren Schnitten in der Hornhaut auszuführen, als dies bei Anwendung von bisher aus dem Stand der Technik bekannten Handstücken der Fall ist. Damit ist dieses Handstück vorteilhaft im Zusammenhang mit medizinisch operativen Verfahren nutzbar, bei denen zum Zwecke der Bildung intraokularer Linsen (IOL) elastisches Material unter die Hornhaut eingebracht wird. Denn Voraussetzung für diese Methode der "injezierbaren intraokularen Linsen" ist die Phacoemulsifikation bei sehr kleinen Schnitten.

## Patentansprüche

1. Medizinisches Instrument zur Laser-Phakoemulsifikation, mit dem das biologische Gewebe einer Augenlinse (2) durch Energieeintrag mittels Laserstrahlung ablatiert, das Ablationsprodukt durch einen Schnitt in der Augenhornhaut (1) abgesaugt und dabei der intraokulare Druck durch Zufuhr einer Irrigationsflüssigkeit aufrecht erhalten wird, umfassend
- zwei Kanülen (3,4) zur Absaugung des ablatierten Linsenmaterials und zur Zufuhr der Irrigationsflüssigkeit sowie mindestens eine Einrichtung zum Energieeintrag durch Laserstrahlung, wobei
- die beiden Kanülen (3,4) von gesonderten, relativ zueinander beweglichen Handstücken (5,6) umschlossen sind, ein Handstück (5) mit der ersten Kanüle (3) über eine Förderleitung (7) mit einer Absaugeinrichtung und ein Handstück (6) mit der zweiten Kanüle (4) über eine weitere Förderleitung (10) mit einer Zuführeinrichtung (11) für die Irrigationsflüssigkeit in Verbindung steht,
**dadurch gekennzeichnet, daß**
- mindestens eines der beiden Handstücke (5,6) mit einem Kupplungsmechanismus für eine Einrichtung zum Zuführen von Substanzen für injezierbare intraokulare Linsen ausgestattet ist, und
- die erste Kanüle (3) und/oder die zweite Kanüle (4) mit Anschlüssen ausgestattet sind, die über den Kupplungsmechanismus mit der Einrichtung zum Zuführen dieser Substanzen in Verbindung stehen.

2. Medizinisches Instrument nach Anspruch 1 , **dadurch gekennzeichnet, daß** jedes der beiden Handstücke und damit jede der beiden Kanülen (3,4) über Förderleitungen sowohl an die Absaugeinrichtung als auch an die Zuführeinrichtung anschließbar sind, wobei in den Förderleitungen zwischen den Kanülen (3,4) und der Absaugeinrichtung bzw. der Zuführeinrichtung ein umschaltbares Wegeventil (16) vorhanden ist, durch das in einer ersten Ventilstellung die erste Kanüle (3) mit der Absaugeinrichtung und die zweite Kanüle (4) mit der Zuführeinrichtung und in einer zweiten Ventilstellung die zweite Kanüle (4) mit der Absaugeinrichtung und die erste Kanüle (3) mit der Zuführeinrichtung verbunden sind.

3. Medizinisches Instrument nach Anspruch 2, **dadurch gekennzeichnet, daß** die erste Kanüle (3) über zwei Förderleitungen (23,24) mit dem Wegeventil (16), die zweite Kanüle (4) über zwei weitere Förderleitungen (27,28) mit dem Wegeventil (16) und das Wegeventil (16) über eine Förderleitung (7) mit der Absaugeinrichtung und über eine weitere Förderleitung (10) mit der Zuführeinrichtung verbunden sind, wobei durch das Wegeventil wahlweise jeweils eine mit der Kanüle (3) verbundene Förderleitung (23) oder eine mit der Kanüle (4) verbundene Förderleitung (28) an die Förderleitung (10) bzw. jeweils eine mit der Kanüle (3) verbundene Förderleitung (24) oder eine mit der Kanüle (4) verbundene Förderleitung (27) an die Förderleitung (7) anschließbar ist.

4. Medizinisches Instrument nach Anspruch 3, **dadurch gekennzeichnet, daß** die Kanülen (3,4) über jeweils einen Anschluß (21,25) verfügen, wobei der Anschluß (21) der ersten Kanüle (3) über eine Verzweigung (22) mit den beiden die Förderleitungen (23,24) und der Anschluß (25) der zweiten Kanüle (4) über eine Verzweigung (26) mit den beiden die Förderleitungen (27,28) verbunden ist.

5. Medizinisches Instrument nach Anspruch 4, **dadurch gekennzeichnet, daß** die Verzweigungen (22,26) in das jeweilige Handstück (5,6) integriert sind.

6. Medizinisches Instrument nach Anspruch 2 bis 5, **dadurch gekennzeichnet, daß** das Wegeventil (16) mit einem Fußschalter verbunden und durch Betätigung dieses Fußschalters wahlweise in die beiden Ventilstellungen umschaltbar ist.

7. Medizinisches Instrument nach einem der vorgenannten Ansprüche, **dadurch gekennzeichnet, daß** jede Kanüle (3,4) einem von zwei gesonderten Handstücken (5,6) zugeordnet ist, wobei beide Handstücke (5,6) relativ zueinander beweglich sind und ergonomisch zur beidhändigen Benutzung durch einen Operateur ausgestaltet sind.

8. Medizinisches Instrument nach einem der vorgenannten Ansprüche, **dadurch gekennzeichnet, daß** mindestens eines der Handstücke (5,6) außer der Kanüle (3,4) die Einrichtung zum Energieeintrag durch Laserstrahlung umfaßt, wobei diese vorzugsweise als Fasertip (13) ausgebildet ist.

9. Medizinisches Handstück nach einem der vorgenannten Ansprüche, **dadurch gekennzeichnet, daß** der Fasertip (13) mindestens abschnittsweise innerhalb der Kanüle (3) und/oder der Kanüle (4) angeordnet ist, wobei die Lichtaustrittsfläche (15) des Fasertips (13) von der Mündungsöffnung (12) der betreffenden Kanüle (3,4) umschlossen ist.

10. Medizinisches Instrument nach einem der vorgenannten Ansprüche, **dadurch gekennzeichnet, daß** der Fasertip (1 3) innerhalb der Kanüle (3,4) in der Strömungsrichtung verschiebbar ist.

11. Medizinisches Instrument nach einem der vorgenannten Ansprüche, **dadurch gekennzeichnet, daß** die erste Kanüle (3) und die zweite Kanüle (4) gleiche Durchmesser im Größenbereich von 0,8 mm bis 1,3 mm haben.

12. Medizinisches Instrument nach einem der Ansprüche 1 bis 10, **dadurch gekennzeichnet, daß** die erste Kanüle (3) und die zweite Kanüle (4) unterschiedliche Durchmesser innerhalb des Größenbereiches von 0,8 mm bis 1,3 mm haben, wobei vorzugsweise die erste Kanüle (3) größer als die zweite Kanüle (4) ausgebildet ist.

13. Medizinisches Instrument nach einem der vorgenannten Ansprüche, **dadurch gekennzeichnet, daß** als Zuführeinrichtung eine Infusionspumpe (11) und als Absaugeinrichtung eine Absaugpumpe (8) vorgesehen sind, die jeweils mit einer steuerbaren Irrigations-/Aspirationseinheit (9) verbunden sind.

14. Medizinisches Instrument nach einem der vorgenannten Ansprüche, **dadurch gekennzeichnet, daß** als Energiequelle ein Er: YAG-Laser vorgesehen ist.

15. Medizinisches Handstück nach einem der vorgenannten Ansprüche, **dadurch gekennzeichnet, daß** das Handstück (5) mit der ersten Kanüle (3) und/oder das Handstück (6) mit der zweiten Kanüle (4) mit Hilfsinstrumenten zum Halten, Greifen, Drehen, Schieben, Schneiden, Spalten, Schützen und/oder Beleuchten des Gewebes am Behandlungsort ausgestattet sind.

16. Medizinisches Instrument nach einem der vorgenannten Ansprüche, **dadurch gekennzeichnet, daß** die Hilfsinstrumente wahl- und wechselweise mittels einer Aufsteckvorrichtung mit den Handstücken (5,6) verbindbar sind.

17. Medizinisches Instrument nach einem der vorgenannten Ansprüche, **dadurch gekennzeichnet, daß** die Hilfsinstrumente an den Handstücken (5,6) versenkbar angeordnet sind.

18. Medizinisches Instrument, nach einem der vorgenannten Ansprüche, **dadurch gekennzeichnet, daß** die Hilfsinstrumente an den Handstücken (5,6) auf einer Führungsbahn verschieblich angeordnet sind.

19. Medizinisches Instrument nach einem der vorgenannten Ansprüche, **dadurch gekennzeichnet, daß** an mindestens einem der beiden Handstücke (5,6) eine Einrichtung zur Reinigung der Kanülen (3,4), der Förderleitungen (7,10,23,24,27,28) und/oder der Verzweigungen (22,26) vorgesehen ist, die bei Bedarf der Beseitigung von Verstopfungen dient.

20. Medizinisches Instrument nach Anspruch 19, **dadurch gekennzeichnet, daß** die Einrichtung zur Reinigung als in Strömungsrichtung verschiebbare Spirale ausgebildet ist.

## Claims

1. A medical instrument for laser phacoemulsification, using which instrument the biological tissue of an ocular lens (2) is ablated by energy input by means of laser radiation, the ablation product is removed by aspiration through a cut in the eye's cornea (1) and, at the same time, the intraocular pressure is maintained by feeding an irrigation liquid, said medical instrument comprising
- two cannulas (3,4) for removing ablated lens material by aspiration and for feeding the irrigation liquid, as well as at least one device for energy input by laser radiation, wherein
- the two cannulas (3,4) are encompassed by separate handpieces (5,6), which are movable relative to each other, a handpiece (5) with the first cannula (3) being in communication with an aspiration device via a feed line (7), and a handpiece (6) with the second cannula (4) being in communication with a feeding device (11) for the irrigation liquid via a further feed line (10),
**characterised in that**
- at least one of the two handpieces (5,6) is provided with a coupling mechanism for a device feeding substances for injectable intraocular lenses, and
- the first cannula (3) and/or the second cannula (4) is/are provided with connections which are in communication with the device for feeding said substances via the coupling mechanism.

2. The medical instrument according to claim 1, **characterised in that** each of the two handpieces and, thus, each of the two cannulas (3,4) is connectable to both the aspiration device and the feeding device via feed lines, with a switchable directional valve (16) being present in the feed lines between the cannulas (3, 4) and the aspiration device or the feeding device, respectively, said valve, when in a first valve position, connecting the first cannula (3) to the aspiration device and, when in a second valve position, connecting the second cannula (4) to the aspiration device and the first cannula (3) to the feeding device.

3. The medical instrument according to claim 2, **characterised in that** the first cannula (3) is connected to the directional valve (16) via two feed lines (23,24), the second cannula (4) is connected to the directional valve (16) via two further feed lines (27,28), and the directional valve (16) is connected to the aspiration device via a feed line (7) and to the feeding device via a further feed line (10), said directional valve selectively or alternately allowing a feed line (23) connected to the cannula (3) or a feed line (28) connected to the cannula (4) to be respectively connected to the feed line (10), or allowing a feed line (24) connected to the cannula (3) or a feed line (27) connected to the cannula (4) to be respectively connected to the feed line (7).

4. The medical instrument according to claim 3, **characterised in that** the cannulas (3,4) have one connection (21,25) each, the connection (21) of the first cannula (3) being connected to both feed lines (23,24) via a branch (22) and the connection (25) of the second cannula (4) being connected to both feed lines (27,28) via a branch (26).

5. The medical instrument according to claim 4, **characterised in that** the branches (22,26) are incorporated into the respective handpiece (5,6).

6. The medical instrument according to claims 2 to 5, **characterised in that** the directional valve (16) is connected to a foot switch, said foot switch allowing the directional valve (16) to be selectively switched to either of the two valve positions.

7. The medical instrument according to any one of the preceding claims, **characterised in that** each cannula (3,4) is associated with one of two separate handpieces (5,6), both handpieces (5,6) being movable relative to each other and being ergonomically designed for two-hand use by an operator.

8. The medical instrument according to any one of the preceding claims, **characterised in that** at least one of the handpieces (5,6) comprises, in addition to the cannula (3, 4), the device for energy input by laser radiation which is preferably provided as a fibre tip (13).

9. The medical handpiece according to any one of the preceding claims, **characterised in that** the fibre tip (13) has at least some portions thereof arranged within the cannula (3) and/or the cannula (4), the light exit surface (15) of the fibre tip (13) being encompassed by the mouth (12) of the respective cannula (3,4).

10. The medical instrument according to any one of the preceding claims, **characterised in that** the fibre tip (13) is displaceable within the cannula (3,4) in the flow direction.

11. The medical instrument according to any one of the preceding claims, **characterised in that** the first cannula (3) and the second cannula (4) have equal diameters ranging from 0.8 mm to 1.3 mm.

12. The medical instrument according to any one of claims 1 to 10, **characterised in that** the first cannula (3) and the second cannula (4) have different diameters ranging from 0.8 mm to 1.3 mm, the first cannula (3) preferably being larger than the second cannula (4).

13. The medical instrument according to any one of the preceding claims, **characterised in that** an infusion pump (11) is provided as the feeding device and an aspiration pump (8) is provided as the aspiration device, each of said pumps being respectively connected to a controllable irrigation / aspiration device (9).

14. The medical instrument according to any one of the preceding claims, **characterised in that** an Er: YAG laser is provided as the energy source.

15. The medical handpiece according to any one of the preceding claims, **characterised in that** the handpiece (5) comprising the first cannula (3) and/or the handpiece (6) comprising the second cannula (4) is/are provided with auxiliary instruments for holding, seizing, rotating, pushing, cutting, splitting, protecting and/or illuminating the tissue at the treatment site.

16. The medical instrument according to any one of the preceding claims, **characterised in that** the auxiliary instruments are selectively and alternately connectable to the handpieces (5,6) by means of a plug-on device.

17. The medical instrument according to any one of the preceding claims, **characterised in that** the auxiliary instruments are retractably arranged on the handpieces (5,6).

18. The medical instrument according to any one of the preceding claims, **characterised in that** the auxiliary instruments are arranged to be displaceable along a guide path on the handpieces (5,6).

19. The medical instrument according to any one of the preceding claims, **characterised in that** at least one of the two handpieces (5,6) is provided with a device for cleaning the cannulas (3,4), the feed lines (7,10,23,24,27,28) and/or the branches (22,26), said device serving to remove occlusions if necessary.

20. The medical instrument according to claim 19, **characterised in that** the cleaning device is provided as a spiral which is displaceable in the flow direction.

## Revendications

1. Instrument médical pour la phaco-émulsification au laser, au moyen de laquelle on procède à l'ablation du tissu biologique d'une lentille oculaire (2) par introduction d'énergie au moyen d'un rayonnement laser, le produit d'ablation étant aspiré par une incision dans la cornée (I) et la pression intraoculaire étant maintenue à cet effet par apport d'un liquide d'irrigation, comprenant
- deux canules (3, 4) pour aspirer la matière de lentille ablatée et pour l'apport du liquide d'irrigation, ainsi qu'au moins un dispositif pour l'introduction d'énergie par rayonnement laser,
- les deux canules (3, 4) étant entourées de pièces manuelles (5, 6) séparées, mobiles l'une par rapport à l'autre, une pièce manuelle (5) avec la première canule (3) étant en liaison par l'intermédiaire d'un conduit de transport (7) avec un dispositif d'aspiration et une pièce manuelle (6) avec la deuxième canule (4) étant en liaison par l'intermédiaire d'un conduit de transport supplémentaire (10) avec un dispositif d'apport (11) pour le liquide d'irrigation, **caractérisé en ce que**
- au moins l'une des deux pièces manuelles (5, 6) est équipée d'un mécanisme de couplage pour un dispositif d'apport de substances pour des lentilles intraoculaires injectables, et
- la première canule (3) et/ou la deuxième canule (4) sont équipées de raccords, qui par l'intermédiaire du mécanisme de couplage sont en liaison avec le dispositif d'apport desdites substances.

2. Instrument médical selon la revendication 1, **caractérisé en ce que** chacune des deux pièces manuelles et donc chacune des deux canules (3, 4) peut se raccorder par l'intermédiaire de conduits de transport, aussi bien sur le dispositif d'aspiration que sur le dispositif d'apport, un distributeur (16) commutable, au moyen duquel dans une première position du distributeur, la première canule (3) est reliée avec le dispositif d'aspiration et la deuxième canule (4) est reliée avec le dispositif d'apport et dans une deuxième position du distributeur, la deuxième canule (4) est en liaison avec le dispositif d'aspiration et la première canule (3) est en liaison avec le dispositif d'apport étant présent dans les conduits de transport, entre les canules (3, 4) et le dispositif d'aspiration ou le dispositif d'apport.

3. Instrument médical selon la revendication 2, **caractérisé en ce que** la première canule (3) est en liaison par l'intermédiaire de deux conduits de transport (23, 24) avec le distributeur (16), la deuxième canule (4) est en liaison par l'intermédiaire de deux autres conduits de transports (27, 28) avec le distributeur (16) et le distributeur (16) est en liaison par l'intermédiaire d'un conduit de transport (7) avec le dispositif d'aspiration et par l'intermédiaire d'un conduit de transport supplémentaire (10) avec le dispositif d'apport, par l'intermédiaire du distributeur, au choix, chaque fois un conduit de transport (23) relié à la canule (3) ou un conduit de transport (28) relié à la canule (4) pouvant se raccorder sur le conduit de transport (10), ou un conduit de transport (24) relié à la canule (3) ou un conduit de transport (27) relié à la canule (4) pouvant se raccorder sur le conduit de transport (7).

4. Instrument médical selon la revendication 3, **caractérisé en ce que** les canules (3, 4) disposent chacune d'un raccord (21, 25), le raccord (21) de la première canule (3) étant relié par l'intermédiaire d'une dérivation (22) avec les deux conduits de transport (23, 24) et le raccord (25) de la deuxième canule (4) étant relié par l'intermédiaire d'une dérivation (26) avec les deux conduits de transport (27, 28).

5. Instrument médical selon la revendication 4, **caractérisé en ce que** les dérivations (22, 26) sont intégrées dans la pièce manuelle (5, 6) respective.

6. Instrument médical selon la revendication 2 à 5, **caractérisé en ce que** le distributeur (16) est relié avec un interrupteur à commande au pied et par manoeuvre de cet interrupteur à commande au pied, il est commutable au choix dans les deux positions du distributeur.

7. Instrument médical selon l'une quelconque des revendications précédentes, **caractérisé en ce que** les deux canules (3, 4) sont associées à l'une de deux pièces manuelles (5, 6) séparées, les pièces manuelles (5, 6) étant mobiles l'une par rapport à l'autre et étant conçues de façon ergonomique, pour l'utilisation par les deux mains d'un opérateur.

8. Instrument médical selon l'une quelconque des revendications précédentes, **caractérisé en ce qu'**au moins l'une des pièces manuelles (5, 6) comprend hormis la canule (3, 4) le dispositif d'introduction d'énergie par rayonnement laser, ce dernier étant conçu de préférence en tant qu'extrémité de fibre optique (13).

9. Pièce manuelle médicale selon l'une quelconque des revendications précédentes, **caractérisé en ce que** l'extrémité de fibre optique (13) est disposée au moins par section à l'intérieur de la canule (3) et/ou de la canule (4), la surface de sortie de lumière (15) de l'extrémité de fibre optique (13) étant entourée par l'orifice d'embouchure (12) de la canule concernée (3,4).

10. Instrument médical selon l'une quelconque des revendications précédentes, **caractérisé en ce que** l'extrémité de fibre optique (13) est déplaçable dans le sens d'écoulement, à l'intérieur de la canule (3,4).

11. Instrument médical selon l'une quelconque des revendications précédentes, **caractérisé en ce que** la première canule (3) et la deuxième canule (4) ont le même diamètre, dans l'ordre de grandeur de 0,8 mm à 1,3 m.

12. Instrument médical selon l'une quelconque des revendications 1 à 10, **caractérisé en ce que** la première canule (3) et la deuxième canule (4) ont des diamètres différents dans l'ordre de grandeur de 0,8 mm à 1,3 mm , la première canule (3) étant de préférence plus grande que la deuxième canule (4).

13. Instrument médical selon l'une quelconque des revendications précédentes, **caractérisé en ce qu'**en tant que dispositif d'apport, on a prévu une pompe à perfusion (11) et en tant que dispositif d'aspiration, on a prévu une pompe aspirante (8) qui sont chacune reliées avec une unité d'irrigation/d'aspiration (9).

14. Instrument médical selon l'une quelconque des revendications précédentes, **caractérisé en ce qu'**on a prévu un laser Er: YAG en tant que source d'énergie.

15. Pièce manuelle médicale selon l'une quelconque des revendications précédentes, **caractérisé en ce que** la pièce manuelle (5) avec la première canule (3) et/ou la pièce manuelle (6) avec la deuxième canule (4) sont équipées d'instruments auxiliaires pour le maintien, la préhension, la rotation, la poussée, la coupe, la scission, la protection et/ou l'éclairage des tissus sur le site de traitement.

16. Instrument médical selon l'une quelconque des revendications précédentes, **caractérisé en ce que** les instruments auxiliaires peuvent être reliés au choix et en alternance au moyen d'un dispositif d'enfichage avec les pièces manuelles (5,6).

17. Instrument médical selon l'une quelconque des revendications précédentes, **caractérisé en ce que** les instruments auxiliaires sont disposés de façon escamotable sur les pièces manuelles (5,6).

18. Instrument médical selon l'une quelconque des revendications précédentes, **caractérisé en ce que** les instruments auxiliaires sont disposés sur les pièces manuelles (5,6) de façon déplaçable sur une glissière de guidage.

19. Instrument médical selon l'une quelconque des revendications précédentes, **caractérisé en ce qu'**un dispositif pour le nettoyage des canules (3, 4), des conduits de transport, (7, 10, 23, 24, 27, 28) et/ou des dérivations (22, 26), destiné en cas de besoin à éliminer les engorgements est prévu sur au moins l'une des deux pièces manuelles (5,6).

20. Instrument médical selon la revendication 19, **caractérisé en ce que** le dispositif de nettoyage est conçu sous la forme d'une spirale déplaçable dans le sens d'écoulement.
